# EUROPEAN PATENT SPECIFICATION

(11) **EP 1 389 063 B1**
(45) Date of publication and mention of the grant of the patent: **08.08.2007**
(21) Application number: 02771591.1
(22) Date of filing: 21.05.2002
(51) Int. Cl.: A61B 7/04, A61B 7/02

(54) **ELECTRONIC MONITORING SYSTEM**
ELEKTRONISCHES ÜBERWACHUNGSSYSTEM
SYSTEME DE CONTROLE ELECTRONIQUE

(30) Priority: 21.05.2001 AU PR515101
(43) Date of publication of application: 18.02.2004
(73) Proprietor: Australian Centre for Advanced Medical Technology, Ltd., Sydney, NSW 2006 (AU)
(72) Inventor: SULLIVAN, Colin, Edward, Birchgrove, New South Wales 2041 (AU)
(74) Representative: Brunner, Michael John
(86) International application number: PCT/AU2002/000615
(87) International publication number: WO 2002/094101

(56) References cited:
- EP-A- 0 467 503
- US-A- 4 528 689
- US-A- 4 720 866
- US-A- 4 991 581
- US-A- 5 150 714
- US-A- 5 213 108
- US-A- 5 701 904

## Description

### Technical Field

This invention concerns an electronic monitoring system for medical use. The system may be used to monitor human and animal patients as well as for non-medical uses where sounds require monitoring over a period of time.

### Background Art

Sounds generated by physiological activity have long been monitored by physicians as an important source of diagnostic information.

The sounds generated by physiological activity arise from a number of sources: breathing, heart operation, digestion and skeletal muscle activation. Many other of the body functions generate sounds, or sub audible vibrations.

Breathing sounds are generated by turbulence as air flows in and out of the lungs. The breathing: sounds may be audible, for instance in the 2-3 kHz range, and they may also extend into the ultrasound range. The turbulence in turn generates low amplitude subtle vibrations within the frequency range of 200 to 900 Hz, but also with lower frequencies in some conditions such as snoring.

Examples of abnormal breathing sounds are snoring and wheezing, the latter typical of asthma, resulting from turbulence when air flows through a narrowed part of a large airway.

Normal heart sounds are generated by closing of the four heart valves, and abnormal sounds (murmurs) occur with turbulence across the valves.

Abnormal gut sounds may arise form the reflux of fluid from the stomach to the oesophagus.

Listening to such sounds plays a central role in the physical examination of a subject and many diseases and abnormalities are identified in part by the hearing of abnormal sounds.

The device which has been used by medical staff to detect both normal and abnormal sounds is the *stethoscope.* This instrument is a physical amplifier, which enables a medical attendant to hear very low volume sounds, and is a principal tool of the physician.

Electronic monitoring systems have been proposed for medical use, which attempt to emulate the physician with a stethoscope by both listening and analysing the sounds heard. Sensitive transducers perform the listening function and they typically produce a high frequency signal in which the sound signals are aliased. Algorithms are then applied to the signal to attempt to interpret the sounds heard.

US 5213108 discloses a stethoscope for use in the ausculation of body sounds. The stethoscope includes a visual display to display the analogue representations of the sounds. The stethoscope is adapted for manipulation and analysis of the received body sounds. Document US-A-5150714 describes an ultrasonic inspection apparatus having a single transducer to record audible signals.

According to the present invention, there is provided an electronic monitoring system for monitoring sounds generated by physiological activity for medical use, comprising:
a first transducer to record at least one channel of audible sound information generated by physiological activity of a subject;
a second transducer to record respiratory movements or heart sounds;
a readout device arranged to display a first visual trace representing a channel of sound information relative to time, and a second visual trace representing either respiratory movement over time or heart sounds over time;
an audio system to selectively replay sound information recorded from a subject and displayed by a visual trace;
where the readout device also displays a cursor which travels over the first and second visual traces as the sound information is replayed to identify the instant on each visual trace that corresponds to the sound heard at the same instant.

Use of the system may make it easier for medical staff to capture and listen to patient sounds of interest. The sounds may be enhanced as described below. The system may also assist trained medical staff to use their skill in analysing the sounds. In other words, rather than seeking to replace the medical staff and their skills, the system seeks to make more efficient use of those hard won skills.

The transducer may be any suitable sound or vibration sensing device, such as those described in other patent applications by the same applicant. They will typically provide an electrical signal modulated with the sounds recorded. Each transducer may produce more than one channel of output, for instance for different frequencies ranges or intensities of sound.

Several of the transducers may be used to record different channels of sound information at the same time. For instance, from different locations on the patient. An array of transducers may be placed in a mattress, or otherwise around a patient for this purpose.

The transducers may also be used in conjunction with other transducers, such as EEG, ECG, video monitoring, temperature or movement monitors.

The readout device will typically comprise a VDU which may be arranged to display several visual traces relating to different recorded signals at the same time. The VDU could also, for example, show a video film of the patient sleeping in part of the screen while the traces are presented in another part of the screen. The display could be reviewed in real time or scrolled back and forward at higher speeds.

Traces of different channels may be displayed one below the other to present the signals synchronized in time. Other traces may represent physiological parameters such as respiratory airflow, or electrocardiogram or pulse etc.

Indicator means may allow a section of the visual trace to be selected. The indicator means could be as simple as a mouse which is manipulated to highlight a section of the visual trace. It will then be possible to replay the sound for that selected section of the trace. The medical staff are able to observe selected traces or other recorded information in order to decide which sections of the trace to highlight and replay. For instance, an increase in temperature or heart rate may indicate that a heart sound should be replayed to ensure normal operation of the heart. A review of the relevant parts of other traces could provide additional information, such as movement occurring, that might tell the medical staff whether or not a replay of the sound was necessary.

The cursor continually tracks the trace, or selected section of the trace, whilst the sound information is played. In this way the medical staff are able to correlate the sound information played on the audio system with the information displayed on the visual trace.

The most widely used way of displaying and recalling physiological events is in the form of a casual event or signal time plot. In this way, events that occur over a period of time can be conceptualised easily, and patterns of event/time recognised. In contrast, to recognise frequencies within sound the information/signal must be heard by the observer in real time. This allows the human brain's superior ability to recognise sound frequencies to be used e.g. the recognition of a voice among many voices in a room full of people.

The purpose of this invention is to provide a method by which both separate capacities of the human brain can examine physiological properties of human function, to enhance the recognition and diagnosis of abnormal events. This is achieved by combining a time series plot (to utilise the human brain's ability to conceptualise the pattern of events) with a replay capacity to allow selected epochs to be played in real time (to utilise the human brain's capacity to process and identify auditory signals).

Thus, a visual display of sound recordings (which may include a range of contributing sources of sound - eg breathing sounds, heart sounds, ambient sounds) does not permit human recognition of the source of those sounds. However, when the epoch of sound is replayed, the human brain can readily distinguish each of these components. The great advantage of such a system is that it does not process the sound information and therefore does not presuppose what is likely to be within the "noise". The end user, by using his/her visual skills can readily identify points in time where sounds of interest have occurred, then quickly and efficiently, by replaying the "sound bite" repeatedly, allow the auditory skills of the brain to identify the nature and origin of the sound. In this way the method allows an end user to see when noise occurred, and then hear and separate the components of noise of interest. Thus, for example an external noise may be generated (say bedroom door slamming, truck passing the house) but in replay this noise will be identifiable, and separable from a patient/subject generated noise of interest (eg the occurrence of wheezing).

Although the same result could possibly be achieved with electronic/computing processing, this would be very complex, requiring the process to "know" every potential noise artefact (eg door slam etc) and every potential pathological sound.

Additionally, the sounds may be filtered and amplified by the audio system to make them easier to hear and interpret. This would enable medical staff to see the filtered trace whilst hearing the corresponding filtered sound. They may also be replayed as many times as is necessary or useful. The audio system may not only play the sounds at real time, but may also play them faster or slower.

The audio system may further provide the capability to auto repeat replay, this capability enabling the medical staff to concentrate on the analysis of the visual trace and sound information.

Electronic processing may also be provided to analyse the recorded information and identify parts of the traces. For instance, after recording information from a patient while they are asleep overnight, the medical staff may wish to review those parts of the traces where the heart rate is elevated above a threshold. The electronics may be able to identify all the periods where this is the case and make them available for replay.

Such processing could be used to alias low and high frequency inaudible sounds to render them audible. Medical staff could then learn to interpret the sounds. For instance, foetal heart sounds are usually hidden behind the mother's heart sounds, but by taking the second harmonic and aliasing it to be audible, it could become useful as a marker for a diagnostic condition.

The electronic processing may also be able to trigger an alarm, for instance if heart sounds stop, or when a particular pathological sound (eg snore, wheeze, cough) occurs.

This combined functionality makes the system useful, not only in a clinical setting such as a hospital, but also for remote observation of patients at home. A nurse or physician may monitor a large number of patients using the system, and be able to respond quickly to any alarm conditions that arise.

In this way, rather than replacing expertise in listening and evaluating the sounds, the system enhances the medical staff skills.

### Brief Description of the Drawings

An example of the invention will now be described with reference to the accompanying drawings, in which:
Figure 1 is a schematic diagram of an example of the system.
Figure 2 is a screenshot of three traces simultaneously displayed by the system of Figure 1 when normal breathing is being observed.
Figure 3 is a screenshot of three traces simultaneously displayed by the system of Figure 1 when obstructive sleep apnoea with reflux is being observed.

### Best Modes of the Invention

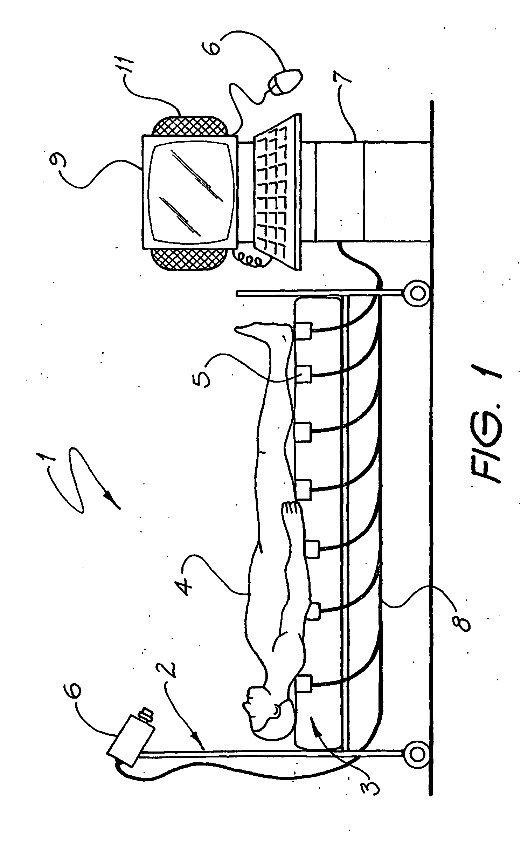

Referring first to Figure 1, the system 1 comprises a bed 2 on which there is a mattress 3 and a patient 4. An array of transducers, one of which is indicated at 5, are placed within the mattress 3 to record sounds and movements of the patient 4. A video camera 6 also films the patient 4.

Output signals from the transducers 5 and the video camera 6 are conducted to the computer 7 by cable 8. The computer 7 displays traces representing the signals collected on its monitor 9.

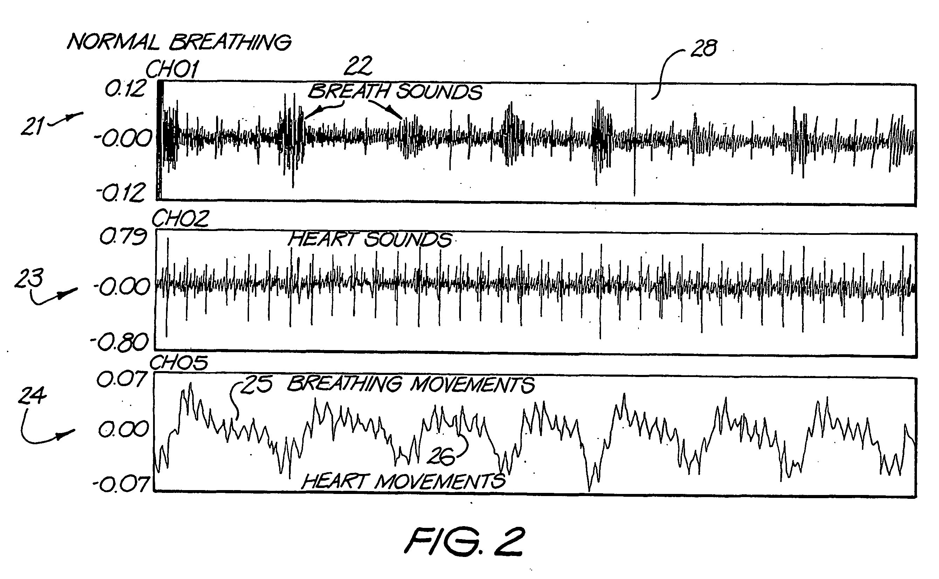

Figure 2 is an example of the traces which could be displayed during normal breathing. In a first channel 21 the trace of breath sounds is displayed. This comprises zero level signal periodically interrupted by high amplitude spikes and at intervals by packets 22 of breathing sounds.

In the second channel 23 are heart sound traces, which can be seen to be the regularly recurring well known heart sound signal.

In the third channel 24 a trace 25 is displayed which represents breathing movements. This trace is reproduced from a movement transducer which is sensitive to vibrations. The overall shape of the trace is a period of elevated amplitude punctuated by low amplitude troughs at the time the breathing sound packets 22 occur. The trace is modulated with heart movements 26.

To facilitate medical staff to locate the exact position on the visual trace that corresponds in time with the sound information, a cursor 28 is displayed on the trace in the first channel 21. The cursor may further traverse channel 23 and channel 24.

It should be appreciated that the three traces shown in the three channels in Figure 2 are synchronised in time. It should also be appreciated that only a few seconds of the signal history are visible on the screen at any given time.

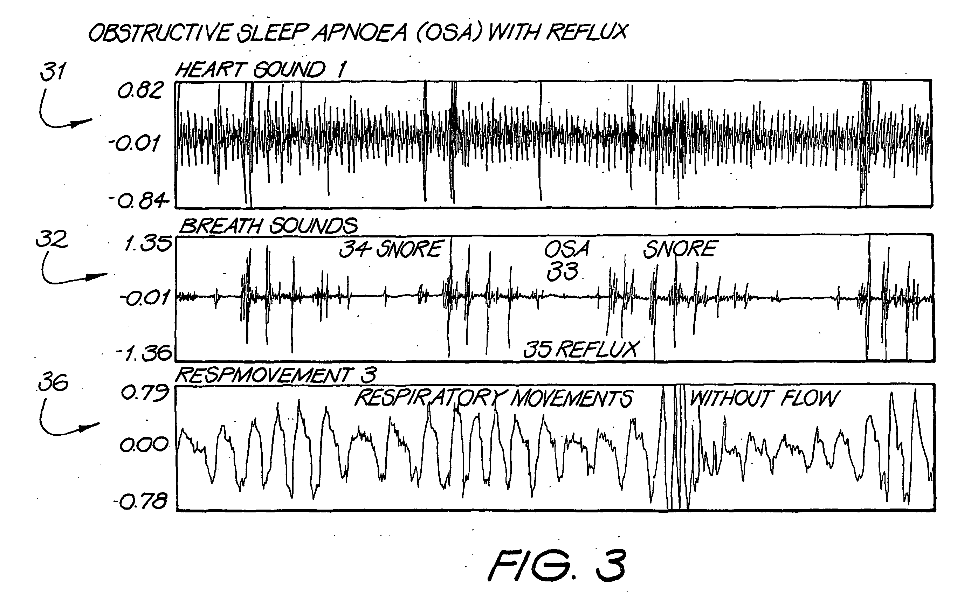

Referring now to Figure 3 we see traces associated with obstructive sleep apnoea with reflux. First it should be noted that the order of the traces has been changed, and now in the first channel 31 the heart sound traces are seen. The scale is slightly different from the scale of channel two 23 in Figure 2, but overall the heart sound traces are elevated with respect to Figure 2.

In the second channel 32 there are breath sound traces and these can be seen to be more spread out than before. At intervals the breath sound is at zero indicating no breathing during periods of obstructive sleep apnoea 33. These periods are punctuated by snores at 34. There is also the occurrence at 35 of reflux sounds.

In the third channel 36 respiratory movement traces can be seen to be continuing throughout the periods of no signal at trace 32, indicating that the breathing muscles are working hard to draw breath during periods of obstructive sleep apnoea where the airways are blocked preventing breath from being drawn.

A mouse 6 associated with a computer can be used to highlight a section of any desired trace, for instance the period around 35 on the second trace 32 can be highlighted on the screen by dragging the mouse over that area. The mouse 6 can further be used to activate an auto repeat replay button on the screen, to enable medical staff to focus on a particular time interval of sound information and corresponding visual trace.

An audio system including speakers 11 may filter and amplify the sounds recorded in trace 32 to remove unwanted sounds and high level signal. The sounds associated with the heart monitor may then be played through the audio system speakers 11 and listened to by the medical staff who, using their skill, will be able to recognise the sounds they are hearing and confirm that reflux is occurring.

Although the invention has been described with reference to a particular example it should be appreciated that many other options are available. For instance, the identification of reflux in an infant may prompt the medical staff to look for bradycardia and apnoea in response to that reflux.

One of the major advantages of this invention is that it is not necessary for the medical staff to continuously observe the patient. In fact a patient with sleep apnoea may be left overnight and monitored using the system. The traces recorded over a period of say eight hours may then be reviewed very quickly the following day by fast forwarding them or by identifying particular epochs of activity which require closer monitoring by the medical staff. These could be identified automatically by algorithms acting on the traces, or they could be identified during fast forward scrolling of the traces by a suitably qualified clinician. For instance fast forward monitoring of the video could identify particular activity which could then be investigated further. On the other hand the video monitor could discount otherwise unusual activity occurring in the traces, for instance if the subject were to sit up during the night and have a coughing fit, it could be seen as not clinically important even though it caused unusual readings from the sensors. This makes efficient use of the medical staffs time in not having to monitor the entire eight hours in real time, but only epochs of interest. In addition it enables the sounds to be heard by the medical staff throughout the epochs of interest and they may be replayed many times if needed.

It should be appreciated that all signals can be transmitted from remote locations to a central monitoring station.

In another form of the invention, the electronic stethoscope is used in conjunction with the electronic monitoring system. The transducer is contained within a sleeve and positioned against the skin of a patient. The transducer produces electrical signals which contain information for visual display of a trace and audio replay of the sound. This signal is then is fed into an input channel in the electronic monitoring system to be stored in the computer for display on the monitor of the computer and simultaneous replay of the sound using speakers.

It will be appreciated by persons skilled in the art that numerous variations and/or modifications may be made to the invention as shown in the specific embodiments without departing from the scope of the invention. The present embodiments are, therefore, to be considered in all respects as illustrative and not restrictive.

## Claims

1. An electronic monitoring system (1) for monitoring sounds generated by physiological activity for medical use, comprising:
a first transducer (5) to record at least one channel of audible sound information generated by physiological activity of a subject;
a second transducer to record respiratory movements or heart sounds;
a readout device (9) arranged to display a first visual trace (21;32) representing a channel of sound information relative to time, and a second visual trace (24;36) representing either respiratory movement over time (24;36) or heart sounds over time (23;31);
an audio system (11) adapted to selectively replay sound information recorded from a subject and displayed by a visual trace
where the readout device is also arranged to display a cursor (28) which travels over the first and second visual traces as the sound information is replayed to identify the instant on each visual trace that corresponds to the sound heard at the same instant.

2. A system according to claim 1, where the transducers (5) are used in conjunction with EEG, ECG, video, temperature or movement monitors to record additional signals.

3. A system according to claim 1 or 2, where the readout device (9) comprises a VDU (9) arranged to display visual traces on its screen relative to respective recorded signals at the same time.

4. A system according to claim 3, where the VDU (9) also shows a video film in part of the screen while the traces are presented in another part of the screen.

5. A system according to claim 3 or 4, where traces of three different channels representing breath sounds (21;32), heart sounds (23;31) and respiratory movement (24;36) are displayed one below the other to present the signals synchronized in time.

6. A system according to any preceding claim, where indicator means (6) allow a section of a visual trace to be selected, and the sound for that selected section of the trace is replayed.

7. A system according to any preceding claim, where the sounds are filtered and amplified.

8. A system according to any preceding claim, where the audio system (11) automatically repeats replay.

9. A system according to any preceding claim, further comprising an electronic processor to analyse the recorded information and identify parts of the traces using algorithms.

10. A system according to any preceding claim, further comprising an electronic processor to alias low and high frequency inaudible sounds to render them audible.

11. A system according to any preceding claim, further comprising an electronic processor to trigger an alarm.

## Patentansprüche

1. Elektronisches Überwachungssystem (1) für die Überwachung von Geräuschen hervorgerufen durch physiologische Aktivität für den medizinischen Gebrauch beinhaltend:
einen ersten Messwandler (5) zum Aufnehmen mindestens eines Kanals hörbarer Geräuschinformation hervorgerufen durch physiologische Aktivität eines Subjektes,
einen zweiten Messwandler zum Aufnehmen von Atmungsbewegungen oder Herzgeräuschen,
eine Auslesevorrichtung (9) eingerichtet zum Anzeigen einer ersten sichtbaren Spur (21; 32), die einen Kanal mit Geräuschinformation im zeitlichen Verlauf repräsentiert, und einer zweiten sichtbaren Spur (24; 36), die entweder Atmungsbewegungen im zeitlichen Verlauf (24; 36) oder Herzgeräusche im zeitlichen Verlauf (23; 31) repräsentiert,
ein Audiosystem (11) eingerichtet zur selektiven Wiedergabe von aufgenommener Geräuschinformation eines Subjekts, die durch eine sichtbare Spur angezeigt wird,
**dadurch gekennzeichnet, dass** die Auslesevorrichtung so eingerichtet ist, dass sie zusätzlich einen Cursor (28) anzeigt, der über die erste und zweite Spur wandert während die Geräuschinformation wiedergegeben wird, um den Moment einer jeden sichtbaren Spur zu identifizieren, der zu dem zu diesem Moment gehörten Geräusch korrespondiert.

2. System nach Anspruch 1, **dadurch gekennzeichnet, dass** die Messwandler (5) in Verbindung mit EEG-, ECG-, Video-, Temperatur oder Bewegungsüberwachung verwendet werden.

3. System nach Anspruch 1 oder 2, **dadurch gekennzeichnet, dass** die Auslesevorrichtung (9) eine Bildschirmeinheit (9) zur gleichzeitigen Anzeige sichtbarer Spuren auf deren Schirm beinhaltet, die zu den aufgenommenen Signalen korrespondieren.

4. System nach Anspruch 3, **dadurch gekennzeichnet, dass** die Bildschirmeinheit (9) zusätzlich in einem Teil des Schirms einen Videofilm zeigt, während die sichtbaren Spuren in einem anderen Teil des Schirms gezeigt werden.

5. System nach Anspruch 3 oder 4, **dadurch gekennzeichnet, dass** Spuren von drei unterschiedlichen Kanälen, die Atemgeräusche (21; 32), Herzgeräusche (23; 31) und Atembewegungen (24; 36) repräsentieren, untereinander angezeigt werden, um die Signale zeitlich synchronisiert anzuzeigen.

6. System nach einem der obigen Ansprüche, **dadurch gekennzeichnet, dass** Anzeigemittel (6) die Auswahl eines Bereichs einer sichtbaren Spur ermöglichen und dass das Geräusch des ausgewählten Bereichs wiedergegeben wird.

7. System nach einem der obigen Ansprüche, **dadurch gekennzeichnet, dass** die Geräusche gefiltert und verstärkt werden.

8. System nach einem der obigen Ansprüche, **dadurch gekennzeichnet, dass** das Audiosystem (11) die Wiedergabe automatisch wiederholt.

9. System nach einem der obigen Ansprüche, **dadurch gekennzeichnet, dass** es zusätzlich einen elektronischen Prozessor zur Analyse der aufgenommenen Information und zur Identifizierung von Bereichen der Spuren unter Verwendung von Algorithmen beinhaltet.

10. System nach einem der obigen Ansprüche, **dadurch gekennzeichnet, dass** es zusätzlich einen elektronischen Prozessor zum Wandeln von nicht hörbaren Geräuschen hoher und niedriger Frequenz enthält, um diese hörbar zu machen.

11. System nach einem der obigen Ansprüche, **dadurch gekennzeichnet, dass** es zusätzlich einen elektronischen Prozessor zur Auslösung eines Alarms beinhaltet.

## Revendications

1. Système de surveillance (1) pour surveiller des sons émis par une activité physiologique, à usage médical, comprenant:
un premier transducteur (5) pour enregistrer au moins un canal d'information sonore audible générée par l'activité physiologique d'un sujet,
un second transducteur pour enregistrer des mouvements respiratoires ou des sons cardiaques,
un dispositif de lecture (9) agencé pour afficher une première trace visible (21; 32) représentant un canal d'information sonore en fonction du temps, et une seconde trace visible (24; 36) représentant soit un mouvement respiratoire en fonction du temps (24; 36), soit des sons cardiaques en fonction du temps (23; 31),
un système audio (11) adapté pour reproduire sélectivement l'information sonore enregistrée pour un sujet et affichée par une trace visible,
dans lequel le dispositif de lecture est également agencé pour afficher un curseur (28) qui se déplace le long de la première et de la seconde traces visibles pendant que l'information sonore est reproduite, pour identifier sur chaque trace visible, l'instant qui correspond au son entendu au même instant.

2. Système selon la revendication 1, dans lequel les transducteurs (5) sont utilisés conjointement avec un appareil de surveillance EEG, ECG, vidéo, de température ou de mouvement, pour enregistrer des signaux supplémentaires.

3. Système selon la revendication 1 ou 2, dans lequel le dispositif de lecture (9) comprend un VDU (9) agencé pour afficher des traces visibles sur son écran, concernant des signaux enregistrés correspondants au même instant.

4. Système selon la revendication 3, dans lequel le VDU (9) montre également un film vidéo dans une portion de l'écran, tandis que les traces sont présentées dans une autre portion de l'écran.

5. Système selon la revendication 3 ou 4, dans lequel des traces de trois canaux différents représentant des sons respiratoires (21; 32), des sons cardiaques (23;31) et un mouvement respiratoire (24; 36) sont affichées l'une en dessous de l'autre pour présenter les signaux synchronisés.

6. Système selon l'une quelconque des revendications précédentes, dans lequel un moyen indicateur (6) permet de choisir une section d'une trace visible, et le son correspondant à la section choisie de la trace est reproduit.

7. Système selon l'une quelconque des revendications précédentes, dans lequel les sons sont filtrés et amplifiés.

8. Système selon l'une quelconque des revendications précédentes, dans lequel le système audio (11) répète automatiquement la reproduction.

9. Système selon l'une quelconque des revendications précédentes, comprenant en outre un processeur électronique pour analyser l'information enregistrée et identifier des portions des traces en utilisant des algorithmes.

10. Système selon l'une quelconque des revendications précédentes, comprenant en outre un processeur électronique pour créneler des sons inaudibles à basse et haute fréquences, pour les rendre audibles.

11. Système selon l'une quelconque des revendications précédentes, comprenant en outre un processeur électronique pour déclencher une alarme
